# EUROPÄISCHE PATENTANMELDUNG

(11) **EP 2 839 772 A1**
(43) Veröffentlichungstag der Anmeldung: **25.02.2015**
(21) Anmeldenummer: 13181548.2
(22) Anmeldetag: 23.08.2013
(51) Int. Cl.: A61B 5/00, A61B 5/22, A63B 21/00, A61B 8/00, A61B 5/0488

(54) **Vorrichtung zur Analyse einer Sehne und zum Widerstandstraining einer Muskel-Sehnen-Einheit**

(71) Anmelder: Mechabionics GmbH & Co. KG, 52068 Aachen (DE)
(72) Erfinder: Oberländer, Kai Daniel, 50931 Köln (DE); Krauss, Peter, 50676 Köln (DE); Fink, Tobias Cornelius, 52064 Aachen (DE); Karamanidis, Kiros, 51427 Bergisch-Gladbach (DE)

(57) **Zusammenfassung**

Die Erfindung betrifft eine Vorrichtung zur Messung/Analyse der mechanischen Eigenschaften, Spannung, Verformung und des Zustandes einer Sehne bzw. Muskel-Sehnen-Einheitund zum Widerstandstraining einer Muskel-Sehnen-Einheit anhand der Sehnendehnungsamplitude, Sehnendehnungsdauer, Sehnendehngeschwindigkeit und Sehnendehnungsfrequenz. Die bekannten Vorrichtungen erlauben keine reproduzierbare Messung mit einer Kontrolle der Verformung, Dehnung und Zugbelastung von Sehnen und/oder Aponeurose. Dies verbessert die Erfindung durch eine Vorrichtung, die zusätzlich eine Aufnahme zur Positionierung des Körperteils, eine Prüfvorrichtung zur Bestimmung der Position des Körperteils relativ zur Aufnahme, wenigstens eine Druckfläche die von dem Körperteil belastbar ist, eine Messvorrichtung zur Vermessung des Zustandes der Sehne und einen Kraftsensor zur Ermittlung der von dem Körperteil auf die Druckfläche aufgebrachten Kraft. Der Kraftsensor kann dann einen ermittelten Kraftwert und die Prüfvorrichtung eine ermittelte Position jeweils in Form eines elektronisch verarbeitbaren Signals ausgeben und eine Datenverarbeitung wertet diese Signale aus und speichert sie ab.

## Beschreibung

Die Erfindung betrifft eine zur Analyse einer Sehne und zum Widerstandstraining einer Muskel-Sehnen-Einheit, insbesondere eine Vorrichtung zur Analyse der mechanischen Eigenschaften, Spannung und Verformung einer Sehne auf Zugbelastung und zum Widerstandstraining einer Muskel-Sehnen-Einheit anhand der Sehnendehnungscharakteristika (Sehnendehnungsamplitude, Sehnendehnungsdauer, Sehnendehnungsgeschwindigkeit bzw. Rate und Sehnendehnungsfrequenz).

Eine Vorrichtung zum Dehnen und Trainieren der Achillessehne ist aus der GB 2 367 763 A bekannt. Bei dieser Vorrichtung wird der Fuß auf eine gegen Federkraft verdrehbare, schiefe Ebene gestellt, die nachfolgend belastet wird. Über Messwertaufnehmer wird dann die maximale Rotation der Ebene gemessen, die der Proband erreichen kann.

Der Nachteil der bekannten Vorrichtung besteht darin, dass diese zwar die Kraft ermitteln kann, die der Proband aufbringen kann, aber keine Analyse des Zustandes und Qualität der Sehne aufweist, so dass der Proband nicht davor geschützt ist, dass die Sehne nicht überbelastet wird, Mikrorupturen entstehen und im schlimmsten Fall, insbesondere bei Vorschädigung, reißen kann oder chronische Entzündungen entstehen können. Ein weiterer Nachteil ist die Tatsache, dass die Messung nicht im hinreichenden Umfang die Besonderheiten des Körpers zu erfassen vermag und insbesondere keine reproduzierbaren Daten ergibt, da durch individuelle anatomische, physiologische und motorische Unterschiede beim Aufstellen und Benutzen der Vorrichtung ein anderes Ergebnis erzielt werden kann.

Darüber hinaus verändern bzw. adaptieren Muskeln und Sehnen unkoordiniert, vor allem während der Wachstumsphase bei Kindern und Jugendlichen, im höheren Lebensalter, nach einer Verletzung, beim Zyklus einer Frau, postmenopausal und durch mechanische Belastung bzw. Immobilisation. Dies führt zu akuten oder langanhaltenden Dysbalancen (Ungleichgewicht) in der Gewebequalität zwischen Muskeln und Sehnen. Eine Bewertung eines Trainings- oder Heilungsfortschritts von Sehnen und Muskelstrukturen, oder Präventionsprogramms zur Vorbeugung von Sehnenverletzungen ist daher nicht möglich. Aufgabe der Erfindung ist es daher, eine Vorrichtung zu schaffen, mit der die mechanischen Eigenschaften, Spannung und Verformung einer Sehne kraft- und dehnungsabhängig und reproduzierbar analysiert werden kann.

Diese Aufgabe wird nach der Erfindung durch eine Vorrichtung zur Analyse der mechanischen Eigenschaften, Spannung und Verformung einer Sehne bei Zugbelastung gelöst, die zusätzlich aufweist:
- eine Aufnahme zur ortsfesten Positionierung zumindest eines Körperteils, das die zu vermessende Sehne und einen damit zusammenwirkenden Muskel enthält,
- eine Prüfvorrichtung zur Bestimmung der Position des Körperteils bzw. Körperteile relativ zur Aufnahme anhand wenigstens zweier anatomischer Punkte des Körperteils,
- wenigstens eine, mit einer vom dem Muskel aufzubringenden Kraft belastbaren Kraftaufnahme,
- einer Messvorrichtung zur Vermessung und Analyse der mechanischen Eigenschaften, Spannung, Verformung und des Zustandes der Sehne und
- zumindest einem Kraftsensor zur Ermittlung der von dem Körperteil auf die insbesondere von einer Druckfläche gebildeten Kraftaufnahme aufgebrachten Kraft, wobei
   der Kraftsensor einen ermittelten Kraftwert und die Prüfvorrichtung eine ermittelte Position jeweils in Form eines elektronisch verarbeitbaren Signals auszugeben vermögen und eine Datenverarbeitung vorgesehen ist, die den Kraftwert in Abhängigkeit der Position des Körperteils abzuspeichern vermag.

Durch die Erfindung kann nun einfach und reproduzierbar die Verformung und mechanische Belastung einer Sehne während einer Muskelkontraktion ermittelt werden und in Abhängigkeit dieser Last und/oder Sehnendehnung entweder trainiert oder die Belastbarkeit der Sehne analysiert werden.

Der Erfindung liegt der Gedanke zugrunde, dass die Sehne und der hiermit verbundene Muskel eine Einheit bilden, die die Kraft auf den Knochen überträgt, wobei Sehne und Muskel zusammen in Reihe geschaltet sind. Wird nun der Muskel kontrahiert, wird die Sehne belastet und infolge der Belastung in die Länge gezogen. Die Bewegung des Verbindungspunktes zwischen dem Muskel und der Sehne kann dann aufgenommen werden, so dass die auf die Sehne wirkende Last bzw. Belastung der einzelnen Fasern der Sehne durch die Messung der Längenveränderung der Sehne gemessen werden kann.

Die Messung der Längenveränderung bzw. die Positionsveränderung des Verbindungspunktes zwischen Sehne und Muskel erfolgt dabei über ein entsprechendes optisches Messverfahren. Dies kann zum Beispiel ein in der Vorrichtung integriertes Ultraschallgerät sein.

Ein weiterer Vorteil kann darin bestehen, dass das Ultraschallgerät lösbar mit der Vorrichtung verwendbar ist, was vor dem Hintergrund, dass ein solches Ultraschallgerät vergleichsweise kostenintensiv ist, die Anschaffung eines zusätzlichen Gerätes oft verhindern kann, da ein Ultraschallgerät in den die Vorrichtung einsetzenden Betrieben, Vereinen, Hochschulen, Praxen bzw. Kliniken häufig vorhanden ist.

Alternativ zu einem Ultraschallgerät können aber auch andere Messverfahren verwendet werden, wobei es zur zuverlässigen Messung notwendig sein wird, einen subkutanen Messpunkt zu erfassen. Theoretisch wäre es allerdings auch möglich, entsprechende äußere Messverfahren anzuwenden, sofern diese mit hinreichend hoher Auflösung eine Erfassung des Übergangspunktes von Muskel zu Sehne oder Knochen zur Sehne und dessen räumlicher Bewegung ermöglichen. So könnte zum Beispiel über zwei neben der Sehne bzw. Muskel angeordnete Elektroden bzw. über eine Mehrzahl solcher Elektrodenpaare, die übereinander angeordnet sind, die Änderung des elektrischen Feldes durch die Längenänderung des Muskels bzw. Sehne ermittelt werden.

Am einfachsten ist es jedoch, mit einer Ultraschalluntersuchung und einer entsprechenden Bildauswertung den Übergang zwischen Sehne und Muskel zu ermitteln.

Grundsätzlich sollte bei der Messung der Belastung der Sehne das Körperteil möglichst reproduzierbar in der Aufnahme gehalten sein. Hierbei ist es auf vielfältige Weise möglich, den Körper der untersuchenden Person zu positionieren. Eine einfache Ausgestaltung der Vorrichtung verwendet hierzu ein Gestell, das einen Grundträger, zum Beispielin Form einer Bodenplatte, aufweist. An diesem Grundträger ist dann ein Rahmen befestigt, der die Aufnahme für das zu vermessende Körperteil aufweist.

Die Aufnahme weist an einer Seite die Druckfläche auf, auf die der Proband bzw. die trainierende Person die von dem Muskel erzeugte Kraft aufbringt. Auf der gegenüberliegenden Seite ist das Widerlager vorgesehen, gegen das sich die Person abstützt. Damit die Aufnahme an die Größe des Körperteils des individuellen Probanden angepasst werden kann, ist der Abstand zwischen der Druckfläche und dem Widerlager einstellbar. Zur Vermeidung von Messfehlern wird dabei bevorzugt das Körperteil spielfrei zwischen dem Widerlager und der Druckfläche eingespannt.

Anstelle der Druckfläche kann auch jede andere Art der Kraftaufnahme verwendet werden. So kann der Proband bzw. die zu trainierende Person die Kraft auch auf einen Umlenkhebel, einen Schwenkarm oder ein Hebelgetriebe aufbringen, so dass zum Beispiel über Drehmomentsensoren die aufgebrachte Kraft anhand des gemessenen Drehmomentes ermittelt werden kann. Dies kann besonders dann hilfreich sein, wenn die Widerlagerfläche relativ zur Person positioniert werden muss, zum Beispiel dann, wenn die Person liegt oder sitzt und die Fläche dann in die geeignete Position gebracht wird.

Damit durch das Einspannen der Proband bzw. die Person nicht verletzt wird, kann eine Kraftbegrenzung vorgesehen sein. Eine solche Kraftbegrenzung ist insbesondere dann sinnvoll, wenn das Einspannen motorisch erfolgt, also entweder die Druckfläche oder das Widerlager motorisch bewegt wird, bis das Körperteil spielfrei in der Aufnahme eingespannt ist.

Nachdem das Körperteil auf diese Weise fest in der Aufnahme gehalten ist, wird erfindungsgemäß die Position des Körperteils relativ zur Vorrichtung ermittelt. Insbesondere wird der Ansatzpunkt des Muskels, der Übergangspunkt zwischen Muskel und Sehne sowie der Endpunkt der Sehne in Bezug auf einen zur Vorrichtung feststehenden Referenzpunkt ermittelt und dokumentiert.

Die Bestimmung dieser Punkte erfolgt erfindungsgemäß über eine Prüfvorrichtung, die die signifikanten Punkte des Körpers aufnimmt. Eine solche Prüfvorrichtung kann zum Beispiel von einem Hebelarm gebildet sein, der mit mehreren, relativ zueinander beweglichen Armsegmenten ausgestattet und schwenkbar am Gehäuse befestigt ist. Dieser Hebelarm kann dann Detektoren für die Drehung in den einzelnen Gelenken aufweisen, die automatisch die Verdrehung der Gelenke erfassen, so dass hierüber die exakte Position, insbesondere des Endes des Hebelarms, automatisch erfasst werden kann.

Die Prüfvorrichtung weist bevorzugt eine Zielvorrichtung auf, über die sie auf die signifikanten Punkte des Körpers, deren Lage aufgenommen werden soll, ausgerichtet werden kann. Eine solche Zielvorrichtung kann zum Beispiel ein Laserstrahl sein, der natürlich im ungefährlichen Bereich arbeitet. Über diesen, zum Beispiel rote Laserstrahlung emittierenden, Laser kann dann der Bediener die Prüfvorrichtung bedienen, indem er den Laserstrahl nacheinander auf die jeweiligen Punkte ausrichtet und jeweils einen Messvorgang auslöst, so dass die Vorrichtung die Position des mit dem Laserstrahl anvisierten Zielpunktes über die Erfassung der jeweiligen Drehwinkel bestimmen und abspeichern kann.

Nachdem auf diese Weise das Körperteil der Person nicht nur bewegungsfrei eingespannt ist, sondern auch in seiner Position sicher bestimmt worden ist, kann nun die Person aufgefordert werden, den jeweils mit der zu messenden Sehne verbundenen Muskel anzuspannen, wodurch eine Kraft auf die Druckfläche ausgeübt wird.

Je nach Zielrichtung des Gerätes kann es natürlich auch möglich sein, dass eine Zugkraft aufzubringen ist. In diesem Fall wird die Funktion der Druckfläche umgekehrt, so dass die Zuglast, die durch den Muskel aufgebracht werden kann, bestimmt wird.

Wird nun die Kraft auf die Druckfläche ausgeübt, wird sich infolge der Kontraktion des Muskels der Übergangspunkt zwischen Muskel und Sehne verlagern, wobei diese Verlagerung über die Messvorrichtung zur Vermessung des Zustandes der Sehne erfasst wird. Dies bedeutet, dass die Messvorrichtung nicht nur den Übergangspunkt zwischen Muskel und Sehne und dessen Lageveränderung aufnimmt, sondern optional insbesondere auch die Änderung der geometrischen Verhältnisse, also insbesondere die Länge und Dicke der Sehne und deren Änderung infolge der Zugbelastung erfasst.

Durch die so vorgenommene Messung kann nun die Verformung und mechanische Belastung der Sehne ermittelt werden. Eine solche Messung hat den besonderen Vorteil, dass die Person, die die Kraft auf die Druckfläche ausübt, vor einer großen Belastung der Sehne bewahrt werden kann (plastischer Bereich der Kraft-Längen- bzw. Spann-Dehnungs-Relation der Sehne). Ein weiterer Vorteil der Vorrichtung besteht darin, dass die Analyse der Materialeigenschaften und der Qualität von Sehnen und Muskeln nicht invasiv möglich wird, was zum Beispiel bei einer Bestimmung der Leistungsfähigkeit eines Sportlers bedeutsam ist.

Ein besonderer Anwendungsfall der Erfindung liegt darin, ein Trainingsprogramm geschaffen wird, bei dem ein Proband, dessen Sehne vorgeschädigt ist oder eine Materialermüdung aufweist, belastungs- und/oder dehnungsabhängig trainieren kann. Hierbei wird im insbesondere dann, wenn die Spannung oder Dehnung einer Sehne einen vorherbestimmten Maximalwert überschreitet, ein Warnsignal ausgegeben, so dass der Proband die Muskelkraft reduzieren kann und damit verhindert, dass seine Sehne geschädigt wird. Eine solche Möglichkeit gibt ihm darüber hinaus das Zutrauen, wieder mit dem Training zu beginnen ohne befürchten zu müssen, dass seine Sehne erneut verletzt wird.

Grundsätzlich ist der Aufbau der Vorrichtung nicht eingeschränkt. Ein möglicher Aufbau besteht darin, dass der Proband bzw. die zu trainierende Person in der Vorrichtung sitzen kann. In diesem Fall weist die Vorrichtung eine Lastaufnahme in Form eines Stuhls bzw. einer Sitzfläche auf. Diese ist vor der Aufnahme für das zu vermessende Körperteil angeordnet, so dass die Person das Körperteil in diese Aufnahme einführen kann, wo es dann zwischen Widerlager und Druckfläche fixiert wird.

Ist zum Beispiel die zu untersuchende Sehne die Achillessehne, wird die Person den unteren Teil des Beins auf die Druckfläche aufstellen, das Widerlager drückt dann auf den Oberschenkel bzw. den oberen Teil des Knies. In diesem Fall kann die Vorrichtung einen U-förmigen Rahmen aufweisen, der auf der Grundplatte montiertist. Die Person sitztvor diesem Rahmen und drückt zur Messung bzw. zum Training die Ferse nach unten auf die Druckfläche.

Alternativ kann, wie oben bereits erwähnt, die Person auch liegen oder auch stehen und die Druckfläche wird über ein andersartiges Gestell so positioniert, dass das gleiche Untersuchungs- bzw. Trainingsprinzip angewendet werden kann. So kann zum Beispiel die Druckfläche auch eine vertikale Fläche sein, gegen die sich die liegende oder stehende Person abstützt, auch hier können die Beine angewinkelt oder auch gestreckt sein, so dass das Widerlager gegen das Knie, Becken, Rücken oder Schulter anstellbar ist. Alternativ kann auch über ein sonstiges Haltemittel das Knie, Becken, Rücken oder Schulter gehalten sein, das dann das Widerlager bildet.

Mit dem erfindungsgemäßen Verfahren können auch alle anderen Sehnen, insbesondere im Bereich des Fuß, Knies, Arms, Fingers, Schulter oder im Halsbereich, vermessen werden, dann wird das Widerlager einen entsprechenden Abschnitt des Körpers abstützen und der Sensor über ein geeignetes Gestänge oder eine sonstiges Halterung auf der anderen Seite des Muskel-/Sehnenstrangs positioniert sein.

Schließlich ist es bei einer besonders bevorzugten Ausgestaltung der Erfindung möglich, über eine Kraftumlenkung mit ein- und demselben Gerät zum Beispiel nicht nur die Achillessehne sondern auch die Patella- oder Quadriceps--Sehne zu messen. Damit die Patella- oder Quadriceps-Sehne gespannt wird, muss die sitzende Person keine Druckkraft nach unten, sondern eine Kraft rechtwinklig zur Vertikalen aufbringen. Insbesondere in diesem Zusammenhang, aber auch bei den anderen Anwendungen, kann die beschriebene Muskel-Sehnen-Diagnostik durch andere Sensoren, zum Beispiel mittels Drehmomentsensoren durchgeführt werden, mit denen die Fähigkeit gemessen wird, ein Drehmoment auf ein Messbauteilaufzubringen. Letztlich ist für die Erfindung nur notwendig, dass, auf welche Weise auch immer, die Kraft direkt oder indirekt bestimmt wird, und die Änderung des Zustandes der Sehne erfasst wird.

Um den gleichen Drucksensor wie bei der Vermessung der Achillessehne verwenden zu können, ist auf der Druckfläche eine kleine Wippe angeordnet, die durch die in horizontale Richtung gerichtete Kraft der Person vergibt wird und mit einem Druckbereich auf die Druckfläche im Bereich des Drucksensors wirkt, so dass das Kippmoment gemessen und als signifikantes Maß für die aufgebrachte Kraft verwendet werden kann. Natürlich sollte die Vorrichtung bei dieser Ausgestaltung eine hinreichende Fixiermöglichkeit für die Kraftumlenkung aufweisen.

Auch bei der Ausgestaltung mit Kraftumlenkung sind alle anderen Anordnungen möglich, es kommt zur Umsetzung der Erfindung lediglich auf die Erkenntnis an, dass das in Reihe geschaltete Paket von Muskel und Sehne geometrisch vermessen wird und dabei die notwendigen Referenzpunkte, um diese Messung reproduzierbar auswerten zu können, hinreichend sicher erfasst werden können.

Ein weiterer Vorteil der Erfindung besteht darin, dass das erfindungsgemäße Gerät transportabel ausgebildet sein kann. Hierzu kann es zum Beispiel einen Rahmen aufweisen, die auf einer Grundplatte montiert ist und entweder demontierbar ist oder schwenkbar mit der Grundplatte derart verbunden ist, so dass er in einer Gebrauchsstellung arretierbar und in eine Transportstellung überführbar ist. Die Lastfläche, also in der Regel der Sitz, auf dem die Person Platz nimmt, kann über ein Gestänge mit der Grundplatte oder dem Rahmen verbunden sein.

Ferner kann die Vorrichtung auch Mittel zur Befestigung an einem handelsüblichen Stuhl oder einer ähnlichen Sitz- bzw. Liegegelegenheit aufweisen, da es lediglich darauf ankommt, dass die Person derart vor der Vorrichtung positioniert werden kann, dass sie das Körperteil in die Aufnahme einführen kann. Diese Aufnahme stellt nicht notwendigerweise einen geschlossenen Raum dar, vielmehr ist mit Aufnahme lediglich gemeint, dass eine Druckfläche und ein Widerlager vorgesehen ist, wobei das Körperteil zwischen beiden Bauteilen bevorzugt spielfrei positioniert werden kann.

Die Lastfläche kann über ein Gestänge mit der Vorrichtung verbunden sein, das bevorzugt an die Körpergröße der Person anpassbar ist. Hierzu kann eine Höhen- und/oder eine Längenverstellung vorgesehen sein.

Schließlich kann im Rahmen einer kinematischen Umkehr die Vorrichtung auch eine mit einer Kraft beaufschlagbaren Druckfläche aufweisen, wobei der Proband oder die zu trainierende Person dann, nachdem das entsprechende Körperteil zwischen Druckfläche und Widerlager positioniert wurde und die Druckfläche mit der Kraft beaufschlagt wird, dieser Kraft entgegenwirken muss. Auch dann entsteht ein entsprechender Belastungszustand, der mit der Vorrichtung ermittelt werden kann.

Die Erfindung betrifft ferner ein Verfahren, bei dem zunächst die mechanischen Eigenschaften, Verformung und Belastung der Sehne ermittelt werden. Diesem Verfahren liegt ebenso wie der Vorrichtung die Erkenntnis zu Grunde, dass Muskel und zugehörige Sehne in Reihe geschaltet sind und zusammen eine gemeinsame Federkonstante besitzen, wobei aus der Bewegung des Übergangspunktes von Muskel zu Sehne oder Knochen zur Sehne die auf die Sehne wirkende Belastung und insbesondere deren Reaktion auf diese Belastung errechnet werden kann. So kann zum Beispiel bei einer kritischen Belastung die Sehne übermäßig verlängert werden, so dass der Übergangspunkt zwischen Sehne und Muskel sehr stark bewegt wird. Ferner wird sich bei sehr großer Belastung die Sehne verlängern und damit im Durchmesser dünner werden, was ebenfalls durch die Messvorrichtung aufgenommen werden kann.

Zur Durchführung des Verfahrens wird der Proband oder die zu trainierende Person derart auf der Lastfläche positioniert, dass sie das Körperteil in die Aufnahme einführen kann, wo es zwischen Widerlager und Druckfläche eingespannt wird. Eingespannt bedeutet in diesem Zusammenhang, dass ohne Schmerzen der Person das Körperteil weitgehend spielfrei gehalten ist.

Um die Untersuchungsergebnisse reproduzierbar aufzeichnen zu können und mit vorherigen Ergebnissen vergleichen zu können, wird bevorzugt über die Prüfvorrichtung zur Bestimmung der Position des Körperteils das in der Aufnahme befindliche Körperteil nochmals vermessen. Hierzu weist die Prüfvorrichtung zum Beispiel im sichtbaren Bereich arbeitende Laserstrahlquellen auf. Bevorzugt sind an beiden Seiten des Körperteils jeweils bewegliche Messarme vorgesehen, an deren Enden oder sonstigen Bereichen eine oder mehrere Laserstrahlquellen vorgesehen sind. Gleichzeitig weisen diese Messarme Mittel auf, die ihre Bewegung und damit ihre räumliche Position erkennen können, so dass die Position und Zielrichtung der beispielsweise als Prüfvorrichtung eingesetzten Laserstrahlquellen ermittelt und abgespeichert werden kann.

Der Bediener wird nun anatomische Stellen des Körperteils der Person mit den Laserstrahlquellen anvisieren. Diese signifikanten Stellen können zum Beispiel spezielle Knochenbereiche oder auch der Übergangsbereich zwischen Sehne und Muskel sein. Auf diese Weise kann die Position des Körperteils innerhalb der Aufnahme sicher ermittelt werden. Gleichzeitig kann das System auf diese Weise lernen, welcher Punkt der Sehne in seiner Bewegung durch die Messvorrichtung, bevorzugt einem Ultraschallgerät, überwacht werden soll.

Der Benutzer wird nun aufgefordert, die Druckfläche zu belasten. Während dieser Belastung wird das in Reihe geschaltete Paket von Sehne und Muskel belastet, so dass sich der Übergangspunkt zwischen Sehne und Muskel bewegen wird. Diese Bewegung kann dann erfasst werden, wobei aus Kraft und Wegstrecke des Übergangspunktes auf den Zustand und Verformung der Sehne und/oder Aponeurose geschlossen werden kann. Bei Erreichen einer kritischen Last, Sehnendehnung oder Sehnenspannung wird bevorzugt ein Warnsignal ausgegeben.

Das oben genannte Verfahren sowie die zugehörige Vorrichtung können zunächst dazu verwendet werden, den Zustand einer Sehne insgesamt in Abhängigkeit der aufgebrachten Kraft zu ermitteln. Dies ist insbesondere zum Beispiel bei Sportlern im Rahmen von Einstellungsuntersuchungen sehr hilfreich.

Insbesondere kann das Verfahren aber auch dazu verwendet werden, nach einer Verletzung die Muskeln und Sehnen zu trainieren und dabei gleichzeitig zu überwachen, ob die Sehne übermäßig bzw. ungünstig belastet wird. Gerade nach einer Verletzung zum Beispiel der Achillessehne hat der Proband (oder Therapeut) oft nicht das notwendige Zutrauen, den Muskel hinreichend zu belasten. Durch das erfindungsgemäße Verfahren wird es nun möglich, ihm eine Überwachungseinrichtung zur Verfügung zu stellen, die ihm den Zustand und die Qualität der Sehne übermittelt, so dass er zunächst das notwendige Selbstvertrauen für das Training wieder erlangen kann und darüber hinaus bei Erreichen eines zu großen Belastungswertes die Trainingsintensität reduzieren kann.

Weitere Merkmale und Vorteile der Erfindung ergeben sich aus den Unteransprüchen und aus der nachfolgenden Beschreibung bevorzugter Ausführungsbeispiele anhand der Zeichnungen.

In den Zeichnungen zeigt:
- Fig. 1: eine erste Ausgestaltung der Erfindung zur Vermessung der Achillessehne in einer dreidimensionalen Ansicht,
- Fig. 2: die Ausgestaltung aus Figur 1 in einer Seitenansicht,
- Fig. 3: die in den Figuren 1 und 2 dargestellte Ausgestaltung der Erfindung mit teilweise zusammengeklapptem Rahmen,
- Fig. 4: eine zweite Ausgestaltung der Erfindung auf Basis der ersten Ausgestaltung, zusätzlich aber mit einer Kraftumlenkung zur Vermessung der Patella- oder Quadriceps-Sehne in einer dreidimensionalen Darstellung und
- Fig. 5: die in Figur 4 dargestellte zweite Ausgestaltung der Erfindung in einer Seitenansicht.

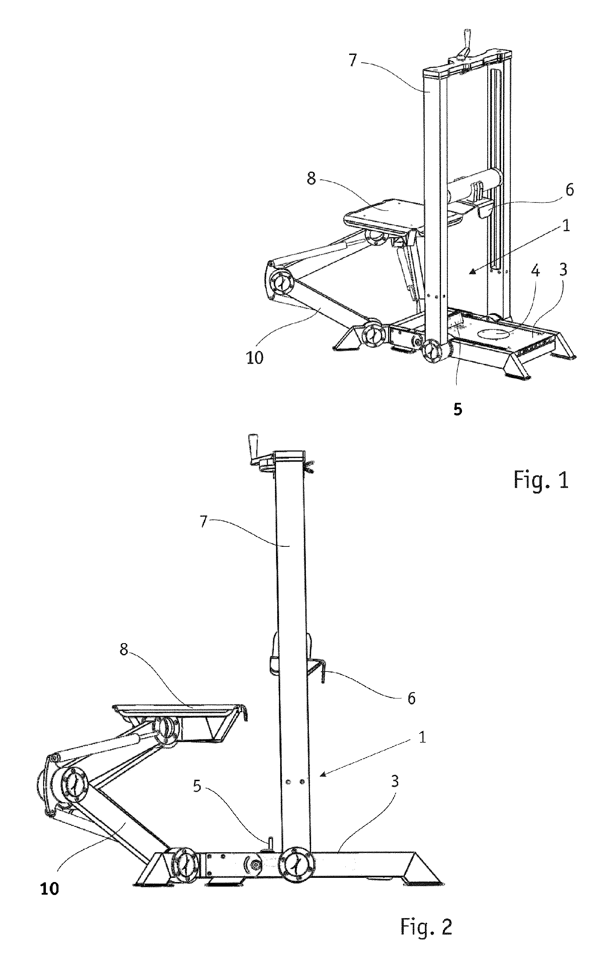

In Figur 1 ist eine Vorrichtung zur Analyse des Zustandes einer Sehne unter Belastung des mit der Sehne verbundenen Muskels dargestellt. Das hier dargestellte Ausführungsbeispiel dient zur Vermessung der Achillessehne. Das in der Vorrichtung zu positionierende Körperteil ist somit der Unterschenkel sowie der Fuß des Probanden bzw. der zu trainierenden Person.

Die Vorrichtung besteht im Wesentlichen aus einer Grundplatte, an der ein Rahmen 7 angeordnet ist. Weiterhin weist die Vorrichtung eine Lastfläche 8 auf, auf der die zu untersuchende oder zu trainierende Person sitzen kann. Die Lastfläche 8 ist relativ zum Rahmen 7 sowohl in der Höhe als auch im Abstand zum Rahmen 7 verstellbar.

Der Rahmen 7 ist über Drehgelenke mit der Grundplatte verbunden und besteht aus zwei im aufgestellten Zustand vertikalen Rahmenelementen, die im oberen Bereich über eine Brücke miteinander verbunden sind. In den Rahmenelementen ist beweglich ein Widerlager 6 geführt, das bevorzugt aber nicht notwendigerweise, über eine Einstellvorrichtung in der Höhe verstellbar ist.

Im dargestellten Ausführungsbeispiel besteht die Einstellvorrichtung aus zwei seitlichen, im Rahmenelement angeordneten Drehspindeln, an denen ein, das Widerlager 6 tragender Querträger gelagertist, wobei über eine im Bereich der Brücke angeordnete Kurbel die Drehspindeln drehbar und damit der Querträger in der Höhe verstellbar ist. Dies ist allerdings nur ein beispielhaftes Ausführungsbeispiel, auch alle anderen möglichen Konstruktionen einer Einstellstellmöglichkeit, bis hin zu einem manuellen Justieren des Widerlagers 6 mit nachfolgender Befestigung, zum Beispiel über Schnellspannschrauben, sind möglich.

Das Widerlager 6 weist einen vorderen Anschlag auf, gegen den die Person ihr Knie anstellen kann. Der Fuß der Person steht auf der hier von einer Druckfläche gebildeten Kraftaufnahme 3, die im hinteren Bereich einen Fersenanschlag 5 aufweist. Unterhalb der Position des Fußes ist der Kraftsensor 4 vorgesehen. Zusätzlich kann ein weiterer Wegsensor 4 vorgesehen sein, der sicherstellt, dass kein Spiel zwischen der Fußsohle und der Kraftaufnahme 3 in Form der Druckfläche vorliegt, bzw. ermöglicht, dass ein eventuelles Spiel größenmäßig erfasst und dann über eine Korrekturrechnung kompensiert werden kann.

Damit die Position des Körperteils, hier also im Wesentlichen des Knies, der Wade und des Fußes reproduzierbar erfasst werden kann, sind (in Figur 1 und 2 nicht dargestellte) Prüfvorrichtungen 2 zur Bestimmung der Position dieses Körperteils vorgesehen. Diese Prüfvorrichtung 2 besteht im gezeigten Ausführungsbeispiel aus zwei Messarmen, die an ihren Enden jeweils eine Laserstrahlquelle aufweisen, die auf signifikante Stellen des Körperteils der Person gerichtet werden kann. Im Einzelnen ist hier die Prüfvorrichtung 2 von zumindest einer Abtastvorrichtung mit der Zielvorrichtung, die auf Stellen des Körperteils ausrichtbar ist, und mit einer Vorrichtung zur Lageerfassung, die die räumliche Bewegung der Zielvorrichtung während des Ausrichtens oder danach zu erfassen und hieraus die Position der jeweiligen Stelle des Körperteils zu ermitteln und als weiterverarbeitbares, elektrisches Signal auszugeben vermag, gebildet.

Sofern anderweitig sichergestellt werden kann, dass sich die signifikanten Punkte des Körperteils der Person immer an der gleichen Stelle befinden, zum Beispiel durch Anschläge und/oder Einspannvorrichtungen, oder es auf die jeweilige Genauigkeit im Einzelfall einmal nicht ankommen sollte, kann auf eine solche Prüfvorrichtung 2 auch verzichtet werden.

Der Sitz ist über ein Sitzgestänge 10 mit der Grundplatte verbunden. Wie auch die Prüfvorrichtung 2 weist das Sitzgestänge 10 ein oder mehrere Gelenke auf, über die es in horizontaler und vertikaler Richtung verstellbar ist. Nach der Einstellung der Sitzposition kann natürlich die als Lastfläche 8 dienende Sitzfläche fixiert werden, alle Gelenke können mit einer Skala versehen sein, so dass die jeweilige Einstellung abgelesen werden und gegebenenfalls später wieder eingestellt werden kann.

Ferner können an den Gelenken, sowohl der Prüfvorrichtung 2 als auch des Sitzgestells 10, elektronische Winkelmesser vorgesehen sein, so dass sich die Winkelstellung automatisch ermitteln lässt und im Steuerprogramm hinterlegt werden kann. Letzteres ist besonders praktisch, wenn die Einstellungen elektrisch oder hydraulisch vorgenommen werden können, so dass die Position einer Person von der Vorrichtung selbsttätig wieder angefahren werden kann.

In Figur 2 ist die Vorrichtung aus Figur 1 in einer Seitenansicht dargestellt. Auch hier ist die Prüfvorrichtung 2 nicht dargestellt, da sie in die Ebene der Grundplatte eingeklappt ist. Zu erkennen ist das Widerlager 6 mit dem vorderen Knieanschlag, das über die obere, am Rahmen 7 befestigte Kurbel in der Höhe verstellbar ist. Zwischen dem Widerlager 6 und der unteren, hier als Druckplatte ausgebildeten Druckfläche 3 ist die Aufnahme 1 zu Positionierung des unteren Teils des Beins der Person vorgesehen. Ein Fersenanschlag 5 erleichtert das Auffinden der geeigneten Fußposition.

Sowohl der Rahmen 6, als auch Sitzgestänge 10 und die hier nicht sichtbare Prüfvorrichtung 2 sind über Gelenke mit der Grundplatte verbunden bzw. weisen im Falle der Prüfvorrichtung 2 und des Sitzgestänge 10 einzelne Segmente auf, die ebenfalls wiederum über Drehgelenke miteinander verbunden sind. Die Winkelpositionen dieser Gelenke kann, wie oben bereits beschrieben, über Skalen oder elektrische Winkelmesser erfasst werden, so dass die Einstellung aller beweglichen Teile, einschließlich des Rahmens 6, abspeicher- und kontrollierbar ist.

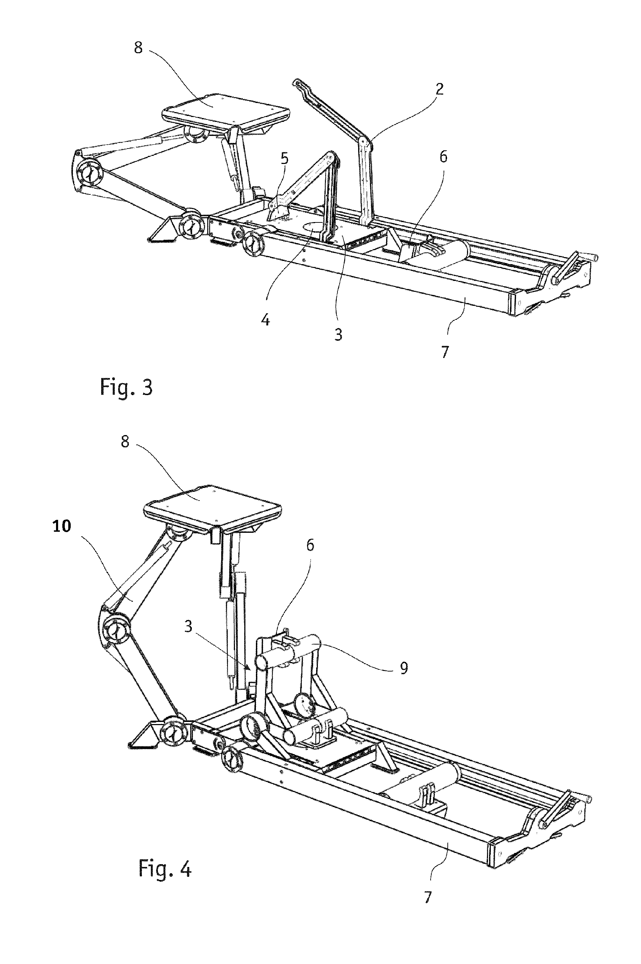

In Figur 3 ist die Vorrichtung nach den Figuren 1 und 2 in einer teilweise zusammengeklappten Position dargestellt. Hier ist der Rahmen 7 nach vorne geklappt, die Prüfvorrichtung 2 wiederum ist ausgeklappt. Ein besonderer Vorzug der hier dargestellten Ausführungsform der Vorrichtung besteht darin, dass sie vollständig zu einem Paket, insbesondere in die Ebene des Rahmens, zusammenlegbar ist. Dies hat den Vorteil, dass sie leicht und einfach transportiert bzw. gelagert werden kann.

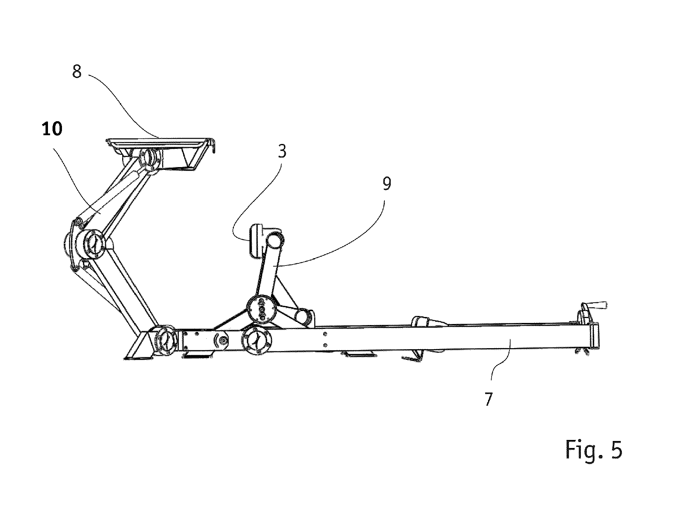

Einen weiteren bevorzugten Aspekt der Erfindung zeigen die Figuren 4 und 5. Hier ist im Wesentlichen die gleiche Vorrichtung gezeigt wie in den Figuren 1-3.

Zusätzlich weist diese Vorrichtung jedoch eine Kraftumlenkung 9 auf. Diese Kraftumlenkung 9 ist auf der Standfläche für den Fuß, die bei der oben beschriebenen Ausgestaltung gemäß der Figuren 1-3 die Kraftaufnahme 3 in Form der Druckfläche bildet, angeordnet. Diese Vorrichtung kann nun zum Analysieren der Patella- oder Quadriceps-Sehne verwendet werden.

Durch die Darstellbarkeit der Lastfläche 8 kann nun die Person so ausgerichtet werden, dass sie mit ihrem Schienbein gegen die vertikale Fläche drückt, so dass das drehbar auf der Fläche aufstehende Kraftumlenkungsteil einem Kippmoment ausgesetzt wird. Dieses Kippmoment wiederum kann von dem Kraftsensor 4 (hier nicht sichtbar) aufgenommen werden, so dass auch hier die von der Person aufgebrachte Kraft ermittelt werden kann.

In allen Fällen ist im hinteren Bereich, bevorzugt unterhalb der Lastfläche 8, ein Ultraschallgerät oder ein sonstiges Mittel zur Erfassung des Zustandes der Sehne vorgesehen. Während der Belastung durch den Druck auf den Kraftsensor 4 kann dieses Kontrollgerät dann den Zustand der Sehne erfassen, insbesondere also die Änderung der Länge und des Durchmessers.

Nicht dargestellt sind in den Figuren die zugehörige Auswerteelektronik sowie das Ultraschallgerät zur Erfassung des Zustandes der Sehne bzw. des Übergangspunktes von Muskel zu Sehne oder Knochen zur Sehne. Ferner weist die Vorrichtung bevorzugt eine Warnvorrichtung auf, die durch ein akustisches oder optisches Signal den beteiligten Personen anzeigt, wenn ein kritischer Last- oder Sehnendehnungszustand erreicht wird. Auch alle anderen optischen Anzeigen, insbesondere Trainingsvorgaben und auch Mindestvorgaben für die aufzubringende Kraft, um einen gewissen Trainings Erfolg zu erzielen, sind möglich.

Ferner kann die Auswerteelektronik das Untersuchungs- und Trainingsergebnis nicht nur auswerten, sondern auch dokumentieren, um so den beteiligten Personen einen Überblick über das erreichte Ergebnis geben zu können.

### Bezugszeichenliste:

- 1: Aufnahme zur Positionierung des Körperteils
- 2: Prüfvorrichtung zur Bestimmung der Position des Körperteils
- 3: Kraftaufnahme
- 4: Kraftsensor
- 5: Fersenanschlag
- 6: Widerlager
- 7: Rahmen
- 8: Lastfläche (Sitz)
- 9: Kraftumlenkung
- 10: Sitzgestänge

## Patentansprüche

1. Vorrichtung zur Analyse der mechanischen Eigenschaften, Spannung und Verformung einer Sehne auf Zugbelastung und zum Widerstandstraining einer Muskel-Sehnen-Einheit anhand der Sehnendehnungsamplitude, Sehnendehnungsdauer, Sehnendehnungsgeschwindigkeit und Sehnendehnungsfrequenz mit
• einer Aufnahme (1) zur ortsfesten Positionierung zumindest eines Körperteils, das die zu vermessende Sehne und einen damit zusammenwirkenden Muskel enthält,
• einer Prüfvorrichtung (2) zur Bestimmung der Position des Körperteils bzw. Körperteile relativ zur Aufnahme (1) anhand wenigstens zweier anatomischer Punkte des Körperteils,
• wenigstens einer, mit einer vom dem Muskel aufzubringenden Kraft belastbaren Kraftaufnahme (3),
• einer Messvorrichtung zur Vermessung und Analyse der mechanischen Eigenschaften, Spannung, Verformung und des Zustandes der Sehne und
• zumindest einem, Sensor, insbesondere einem Kraftsensor (4) und/oder einem Drehmomentsensor zur direkten oder indirekten Ermittlung der von dem Körperteil auf die Kraftaufnahme (3) aufgebrachten Kraft oder des Drehmoments, wobei
der Sensor einen ermittelten Kraftwert und die Prüfvorrichtung (2) eine ermittelte Position jeweils in Form eines elektronisch verarbeitbaren Signals auszugeben vermögen und eine Datenverarbeitung vorgesehen ist, die den Kraftwert in Abhängigkeit der Position des Körperteils abzuspeichern vermag.

2. Vorrichtung zur Analyse der mechanischen Eigenschaften, Spannung, Verformung und des Zustandes einer Sehne bzw. Muskel-Sehnen-Einheit unter Belastung nach Anspruch 1, **dadurch gekennzeichnet, dass** die Aufnahme (1) ein Widerlager (6) aufweist, wobei zwischen dem Widerlager (6) und der Kraftaufnahme (3) das Körperteil positionierbar ist und der Abstand zwischen dem Widerlager (6) und der insbesondere von einer Druckfläche gebildeten Kraftaufnahme (3) zur Anpassung an die Abmessung des Körperteils veränderbar ist.

3. Vorrichtung zur Analyse der mechanischen Eigenschaften, Spannung, Verformung und des Zustandes einer Sehne bzw. Muskel-Sehnen-Einheit unter Belastung nach Anspruch 1 oder 2, **dadurch gekennzeichnet, dass** sie eine zusätzliche Lastaufnahme (8) aufweist, auf der der Körper während der Durchführung der Messung zu ruhen vermag, wobei die Lastaufnahme (8) insbesondere von einem Sitz oder eine Liegefläche gebildet ist.

4. Vorrichtung zur Analyse der mechanischen Eigenschaften, Spannung, Verformung und des Zustandes einer Sehne bzw. Muskel-Sehnen-Einheit unter Belastung nach einem der vorhergehenden Ansprüche, **dadurch gekennzeichnet, dass** die Prüfvorrichtung (2) zur Bestimmung der Position des Körperteils von zumindest einer Abtastvorrichtung mit einer Zielvorrichtung, die auf Stellen des Körperteils ausrichtbar ist, und mit einer Lageerfassung, die die räumliche Bewegung der Zielvorrichtung während des Ausrichtens zu erfassen vermag und hieraus die Position der jeweiligen Stelle des Körperteils zu ermitteln und als weiterverarbeitbares, elektrisches Signal auszugeben vermag, gebildet ist.

5. Vorrichtung zur Analyse der mechanischen Eigenschaften, Spannung, Verformung und des Zustandes einer Sehne bzw. Muskel-Sehnen-Einheit unter Belastung nach dem vorhergehenden Anspruch, **dadurch gekennzeichnet, dass** die Zielvorrichtung eine optische Zielerfassung in Form eines, insbesondere von einem Laserstrahl gebildeten, Zielpunkts aufweist, der auf die Stelle des Körperteils ausrichtbar ist, wobei die Zielerfassung ein oder mehrere Verstellmöglichkeiten in Form von Gelenken und/oder Teleskopstangen aufweist und die Verstellmöglichkeiten mit Wegund/oder Winkelerfassungsmitteln versehen sind, so dass der Weg der Zielerfassung bis zum Auftreffen auf die Stelle des Körperteils reproduzierbar relativ zu einem Referenzpunkt an der Vorrichtung erfassbar ist.

6. Vorrichtung zur Analyse der mechanischen Eigenschaften, Spannung, Verformung und des Zustandes einer Sehne bzw. Muskel-Sehnen-Einheit unter Belastung nach Anspruch 2, **dadurch gekennzeichnet, dass** im Bereich der Kraftaufnahme (3) ein Wegsensor vorgesehen ist, der zusätzlich zur Kraftmessung zur Kompensation eines eventuell zwischen dem Widerlager (6) und Kraftaufnahme (3) verbliebenen Spiels eine Wegstrecke während des Aufbringens der Druckkraft zu messen und an die Datenverarbeitung weiterzugeben vermag.

7. Vorrichtung zur Analyse der mechanischen Eigenschaften, Spannung, Verformung und des Zustandes einer Sehne bzw. Muskel-Sehnen-Einheit unter Belastung nach einem der vorhergehenden Ansprüche, **dadurch gekennzeichnet, dass** die Messvorrichtung zur Vermessung des Zustandes der Sehne von einem Bilderfassungs- oder Ultraschallmessgerät gebildet ist, dass die Verformung (Länge und Dicke) der Sehne und/oder Aponeurose während des Aufbringens der Druckkraft auf die Kraftaufnahme (3) optisch zu ermitteln vermag, wobei eine Bildverarbeitung aus der Länge und/oder Dicke der Sehen ein Belastungssignal ermittelt und an die Datenverarbeitung weitergibt.

8. Vorrichtung zur Analyse der mechanischen Eigenschaften, Spannung, Verformung und des Zustandes einer Sehne bzw. Muskel-Sehnen-Einheit unter Belastung nach dem vorhergehenden Anspruch, **dadurch gekennzeichnet, dass** die Datenverarbeitung derart ausgestaltet ist, dass sie bei Erreichen eines insbesondere einstellbaren Grenzwertes, der die Verformungsamplitude der Sehne repräsentiert, einen kritischen Belastungszustand annimmt und ein entsprechendes Signal auszugeben vermag.

9. Vorrichtung zur Analyse der mechanischen Eigenschaften, Spannung, Verformung und des Zustandes einer Sehne bzw. Muskel-Sehnen-Einheit unter Belastung nach einem der vorhergehenden Ansprüche, **dadurch gekennzeichnet, dass** sie zur Messung der Belastung der menschlichen Achillessehne ausgebildet ist, wobei auf die Kraftaufnahme (3) der Fuß des Menschen aufsetzbar ist und das Widerlager (6) derart ausgebildetist, dass es auf den Oberschenkel oder das Knie des Menschen drückt.

10. Vorrichtung zur Analyse der mechanischen Eigenschaften, Spannung, Verformung und des Zustandes einer Sehne bzw. Muskel-Sehnen-Einheit unter Belastung nach dem vorhergehenden Anspruch, **dadurch gekennzeichnet, dass** die Kraftaufnahme (3) von einer Platte gebildet ist, die am hinteren Ende einen aus der Kraftaufnahme (3) hervorspringenden Fersenanschlag (5) aufweist.

11. Vorrichtung zur Analyse der mechanischen Eigenschaften, Spannung, Verformung und des Zustandes einer Sehne bzw. Muskel-Sehnen-Einheit unter Belastung nach einem der vorhergehenden Ansprüche, **dadurch gekennzeichnet, dass** die Vorrichtung einen Rahmen (7) aufweist, in den das Körperteil einbringbar ist, wobei der Rahmen (7) im unteren Bereich die als Trittplatte ausgebildete Kraftaufnahme (3) und im oberen Bereich das Widerlager (6) aufweist und mit dem Rahmen (7) auf einer Seite die als Sitz ausgebildete und insbesondere in Höhe und/oder Abstand zum Rahmen verstellbare Lastaufnahme (8) angeordnet ist, die über ein Gestänge mit dem Rahmen (7) verbunden ist.

12. Vorrichtung zur Analyse der mechanischen Eigenschaften, Spannung, Verformung und des Zustandes einer Sehne bzw. Muskel-Sehnen-Einheit unter Belastung nach dem vorhergehenden Anspruch, **dadurch gekennzeichnet, dass** der Rahmen (7) und das Gestänge mit der Lastaufnahme (8) zu einem ebenen Paket zusammenlegbar sind, wobei hierzu der Rahmen (7) arretierbar und schwenkbar an einer Grundplatte befestigt ist und das Gestänge arretierbar und schwenkbar an der Grundplatte oder an dem Rahmen (7) befestigt ist.

13. Vorrichtung zur Analyse der mechanischen Eigenschaften, Spannung, Verformung und des Zustandes einer Sehne bzw. Muskel-Sehnen-Einheit unter Belastung nach einem der vorhergehenden Ansprüche, **dadurch gekennzeichnet, dass** die Vorrichtung eine auf der Kraftaufnahme (3) anordnenbare Kraftumlenkung (9) aufweist, die einen auf die Kraftaufnahme (3) aufstellbaren und hiermit befestigbaren Träger und einen Anschlag in einer zur Oberfläche der Kraftaufnahme (3) parallelen Richtung aufweist, gegen den das Körperteil pressbar ist, wobei die Kraftumlenkung (9) die parallel zur Kraftaufnahme (3) gegen den vorderen Anschlag gerichtete Kraft in eine auf die Kraftaufnahme (3) wirkende Druckkraft umzuwandeln vermag und hierzu der Anschlag drehbar an dem Träger angeordnet ist, so dass die gegen den Anschlag wirkende Kraftin eine tangentiale Kraft umgewandelt wird.

14. Vorrichtung zur Analyse der mechanischen Eigenschaften, Spannung, Verformung und des Zustandes einer Sehne bzw. Muskel-Sehnen-Einheit unter Belastung nach Anspruch 2 oder einem der Ansprüche 3 bis 13 in Verbindung mit Anspruch 2, **dadurch gekennzeichnet, dass** das Widerlager (6) in der Position relativ zum Körperteil verstellbar ist und einen Kraftsensor aufweist, der die von dem Widerlager (6) auf das Körperteil bei Anstellen des Widerlagers (6) an das Körperteil wirkende Kraft zu ermitteln vermag, wobei das Widerlager (6) motorisch bewegt ist und eine Kraftbegrenzung vorgesehen ist, die bei Erreichen eines definierten Grenzwertes der von dem Widerlager (6) auf das Körperteil aufgebrachten Kraft die weitere Bewegung des Widerlagers (6) beendet und das Widerlager (6) räumlich arretiert.

15. Verfahren zur Messung der mechanischen Eigenschaften, Spannung, Verformung und des Zustandes einer Sehne bzw. Muskel-Sehnen-Einheit und zum Training eines mit einer Sehne zusammenwirkenden Muskels mittels einer Vorrichtung zur Analyse des Zustandes einer Sehne unter Belastung nach einem der vorhergehenden Ansprüche, **dadurch gekennzeichnet, dass** zunächst die Person in der Aufnahme (1) positioniert wird, anschließend das, die zu messende Sehne und/oder Aponeurose enthaltende Körperteil auf der Kraftaufnahme (3) ausgerichtet und das Körperteil über das Widerlager (6) derart fixiert wird, dass in Richtung der von dem mit der Sehne verbundenen Muskel aufgebrachten Kraft das Körperteil zwischen Kraftaufnahme (3) und Widerlager (6) eingespannt ist, wobei die Lage signifikanter Punkte des Körperteils, insbesondere signifikanter Punkte von Knochen und/oder des Übergangs von Muskel zu Sehne oder Knochen zur Sehne über die Prüfvorrichtung (2) detektiert und abgespeichert wird, und wobei die Person nachfolgend aufgefordert wird, die Kraftaufnahme (3) mit einer Kraft zu belasten und über die Messvorrichtung die Verformung und/oder Dicke der Sehne sowie deren Verformung (Längen- und/oder Dickenänderung) und/oder die räumliche Lage des Übergangspunktes zwischen Muskel und Sehne oder Knochen zur Sehne und dessen Bewegung, jeweils in Abhängigkeit der aufgebrachten Kraft, ermittelt und abgespeichert wird, wobei insbesondere aus dem Ergebnis der Messvorrichtung die Sehnendehnung und Belastung errechnet wird und bei Erreichen eines kritischen Zustandes ein Warnsignal ausgegeben wird.
